# EUROPÄISCHE PATENTANMELDUNG

(11) **EP 2 438 891 A1**
(43) Veröffentlichungstag der Anmeldung: **11.04.2012**
(21) Anmeldenummer: 11180154.4
(22) Anmeldetag: 06.09.2011
(51) Int. Cl.: A61F 2/90

(54) **Stent mit erhöhter Sichtbarkeit im Röntgenbild**

(30) Priorität: 08.10.2010 US 391087 P
(71) Anmelder: Biotronik AG, 8180 Bülach (CH)
(72) Erfinder: Fircho, Horst, 18184 Kösterbeck (DE); Bakczewitz, Frank, 18055 Rostock (DE); Wolfer, Markus, 8451 Kleinandelfingen (CH)
(74) Vertreter: Lindner-Vogt, Karin L.

(57) **Zusammenfassung**

Die Erfindung betrifft einen Stent umfassend einen röhrenförmigen Grundkörper mit einem Lumen entlang einer Längsachse, wobei der Grundkörper eine Mehrzahl von umlaufenden Stützstrukturen und einen oder mehrere Konnektoren aufweist, wobei zwei aufeinanderfolgende umlaufende Stützstrukturen über mindestens einen Konnektor miteinander verbunden sind, dadurch gekennzeichnet, dass eine, mehrere oder alle Stützstrukturen und/oder Konnektoren einen schlitzförmigen Durchbruch aufweisen, wobei der schlitzförmige Durchbruch mit einem röntgendichten Material gefüllt ist und eine Krümmung aufweist, die mindestens zu einem Ende des schlitzförmigen Durchbruchs hin zunimmt.

## Beschreibung

Die Implantation von Stents hat sich als eine der wirkungsvollsten therapeutischen Maßnahmen bei der Behandlung von Gefäßerkrankungen etabliert. Stents haben den Zweck, in Hohlorganen eines Patienten eine Stützfunktion zu übernehmen. Stents herkömmlicher Bauart weisen dazu einen Grundkörper auf, der eine Vielzahl von umlaufenden Stützstrukturen z. B. aus metallischen Streben aufweist, der zur Einbringung in den Körper zunächst in einer komprimierten Form vorliegt und am Ort der Applikation aufgeweitet wird. Einer der Hauptanwendungsbereiche solcher Stents ist das dauerhafte oder temporäre Weiten und Offenhalten von Gefäßverengungen, insbesondere von Verengungen (Stenosen) der Herzkranzgefäße. Daneben sind beispielsweise auch Aneurysmenstents bekannt, die zur Stützung beschädigter Gefäßwände dienen.

Stents besitzen einen rohrförmigen Grundkörper von ausreichender Tragkraft, um das verengte Gefäß im gewünschten Maße offen zu halten, durch den der Blutfluss ungehindert weiterläuft. Die Umfangswandung des Grundkörpers wird in der Regel von einer gitterartigen Stützstruktur gebildet, die es erlaubt, den Stent in einem komprimierten Zustand mit kleinem Außendurchmesser bis zur zu behandelnden Engstelle des jeweiligen Gefäßes einzuführen und dort beispielsweise mit Hilfe eines Ballonkatheters soweit aufzuweiten, dass das Gefäß den gewünschten, vergrößerten Innendurchmesser aufweist. Der Vorgang der Positionierung und Expansion der Stents während der Prozedur und die abschließende Lage des Stents im Gewebe nach Beendigung der Prozedur muss durch den Kardiologen überwacht werden. Dies kann durch bildgebende Verfahren, wie z. B. durch Röntgenuntersuchungen erfolgen.

Meist besteht der Stent selbst aus Werkstoffen, die nicht ausreichend röntgendicht sind, um durch eine bildgebende Röntgenuntersuchung mit der gewünschten Qualität darstellbar zu sein. D. h. damit ein Stent im Röntgenbild darstellbar ist, wird der Stent meist mit einem röntgendichten Material, einem geeigneten Röntgenmarker, versehen.

Derzeit werden solche Röntgenmarker meist entweder als endständige, flächige Beschichtungen auf den Stent aufgetragen oder punktförmig, z. B. in einem Mikronietverfahren, an dem Stent befestigt. Dabei werden röntgendichte Materialien verwendet, die z. B. Au, Pt und/oder Ta beinhalten oder daraus bestehen. Diese Art der Markierung von Stents mit röntgendichten Markern hat den Nachteil, dass die Röntgensichtbarkeit relativ gering ist und meist stark vom Strahlengang beeinflusst wird und damit lageabhängig ist.

Aufgrund der geringen Schichtdicke ist die Röntgensichtbarkeit insbesondere bei endständig, flächig beschichteten Stents relativ gering. Zudem treten in der Angiographie sogenannte "dog boning"-Effekte auf, d. h. die Enden überstrahlen das Bild derartig, dass der Eindruck entsteht, die Stentenden seien weiter geöffnet als der mittlere Stentbereich. Dazu kommt, dass in den Bereichen des Stents, die einer Verformung ausgesetzt sind, die Gefahr besteht, dass sich die Beschichtung mit dem Röntgenmarker ablöst und verloren geht.

Das Mikronietverfahren ist dagegen apparativ aufwändig, kostenintensiv und kann zu einer nicht zu vernachlässigenden Ausschussrate führen. Zudem muss die Stentarchitektur und das Crimpdesign so gewählt werden, dass die Stentenden ausreichend Platz für die Aufnahme der zylinderförmigen Nieten bieten.

Aufgabe der vorliegenden Erfindung ist es einen oder mehrere der Nachteile des Standes der Technik zu vermindern oder zu vermeiden.

Die Aufgabe wird gelöst durch Bereitstellung eines Stents umfassend einen röhrenförmigen Grundkörper mit einem Lumen entlang einer Längsachse, wobei der Grundkörper eine Mehrzahl von umlaufenden Stützstrukturen und einen oder mehrere Konnektoren aufweist, wobei zwei aufeinanderfolgende umlaufende Stützstrukturen über mindestens einen Konnektor miteinander verbunden sind, dadurch gekennzeichnet, dass eine, mehrere oder alle Stützstrukturen und/oder Konnektoren einen schlitzförmigen Durchbruch aufweisen, wobei der schlitzförmige Durchbruch mit einem röntgendichten Material gefüllt ist und eine Krümmung aufweist, die mindestens zu einem Ende des schlitzförmigen Durchbruchs hin zunimmt.

In die Bestandteile der Grundstruktur des erfindungsgemäßen Stents, insbesondere in die Stützstrukturen und/oder Konnektoren, werden schlitzförmige Durchbrüche eingebracht, die den Steg der Stützstrukturen und/oder Konnektoren von der Außenoberfläche bis zur Innenoberfläche durchdringen. Diese schlitzförmigen Durchbrüche sind mit einem röntgendichten Material gefüllt. Ähnlich wie bei der Verwendung von Mikronieten, wird durch das Anbringen von im Vergleich zur endständigen Beschichtung größeren Mengen an röntgendichtem Material eine sehr hohe Röntgensichtbarkeit erreicht. Im Gegensatz zur Verwendung von flächigen Beschichtungen ist die Kontaktfläche zwischen röntgendichtem Material und Gefäßwand oder Blutstrom sehr klein. Der schlitzförmige Durchbruch weist eine Krümmung auf, die nicht über die gesamte Länge des Schlitzes gleich ist, sondern mindestens zu einem Ende des schlitzförmigen Durchbruchs hin zunimmt. Damit befindet sich das röntgendichte Material nicht nur über eine vergleichsweise größere Fläche in Kontakt mit dem Material des Grundkörpers des Stents, sondern das röntgendichte Material wird auch durch die verschiedenen Krümmungen des schlitzförmigen Durchbruchs mechanisch besser fixiert.

Dadurch dass das röntgendichte Material im schlitzförmigen Durchbruch gleichzeitig durch Kraft- und Formschluss fixiert ist, ist also eine, auch im Vergleich zur Verwendung von Mikronieten, besonders stabile Einbindung des röntgendichten Materials im Stent gewährleistet, so dass die Wahrscheinlichkeit des Verlustes von Röntgenmarkermaterial während der Verwendung des Stents deutlich reduziert ist. Neben der mechanischen Stabilität, zeichnet sich der erfindungsgemäße Stent dadurch aus, dass dieser nach weniger aufwändigen Verfahren wie Laserschnitt und Abscheidung von röntgendichtem Material hergestellt werden kann und so der hohe Aufwand und Ausschussrate des Mikronietenverfahrens vermieden werden kann.
Die bogenförmig gekrümmte Geometrie der Marker hat zudem den Vorteil, die Lageabhängigkeit anderer Markergeometrien (bspw. Niet) zu verringern, indem in allen Lagesituationen eine hohe Markerdicke und damit Absorption der Röntgenstrahlen vorhanden ist, wodurch die Sichtbarkeit verbessert wird.

Die schlitzförmigen Durchbrüche können grundsätzlich an jeder Stelle des Grundkörpers des Stents angebracht sein. Das hat gegenüber endständigen Markern (typisch bei Nieten und flächenhaften Beschichtungen) den Vorteil, dass der Stent bei Einsatz im Bereich von Bifurkationen sehr genau positioniert werden kann, da durch eine geeignete Anordnung der Schlitzmarker im mittleren Längenbereich des Stents die Stentposition bzgl. der Abzweigung in der Angiographie genau ermittelt werden kann.

Der erfindungsgemäße Stent weist einen röhrenförmigen Grundkörper auf, der ein Lumen entlang einer Längsachse des Stents umschließt. Durch dieses Lumen kann, nach erfolgter Applikation des Stents in ein Blutgefäß, ein Blutfluss erfolgen.

Der Grundkörper des erfindungsgemäßen Stents umfasst eine Mehrzahl von umlaufenden Stützstrukturen, die aufeinanderfolgend entlang der Längsachse des Stents angeordnet sind und das Lumen umschließen. Die Stützstrukturen sind jeweils aus einer Aneinanderreihung von Diagonalelementen und Bogenelementen (auch Kronen genannt) aufgebaut, die jeweils aus Streben aus einem Implantatwerkstoff gebildet sein können. Die Diagonalelemente weisen eine langgestreckte Form mit zwei Enden auf und verbinden zwei Bogenelemente mit gegenläufig ausgerichteter Krümmung. Die Diagonalelemente sind im Wesentlichen für die Ausdehnung einer Stützstruktur in längsachsialer Richtung verantwortlich. Die Bogenelemente sind gekrümmt und verbinden zwei aufeinanderfolgende Diagonalelemente einer Stützstruktur derart miteinander, dass diese entlang einer zur Längsachse vertikal verlaufenden Achse übereinander zu liegen kommen, wobei eine ringförmige umlaufende Struktur entsteht, die ein Lumen umschließt.

Der Grundkörper des erfindungsgemäßen Stents umfasst neben einer Mehrzahl von Stützstrukturen einen oder mehrere Konnektoren, wobei zwei aufeinander folgende umlaufende Stützstrukturen über mindestens einen Konnektor miteinander verbunden sind. Einerseits müssen diese Konnektoren so angeordnet sein, dass sie eine ausreichende Biegeflexibilität des Stents gewährleisten, andererseits dürfen sie einen Crimp- und/oder Dilatationsprozess nicht behindern. Die Konnektoren des erfindungsgemäßen Stents sind derart ausgestaltet, dass eine Mehrzahl von Stützstrukturen zu einem Grundkörper verbunden werden können, der für den Einsatz in einem expandierbaren Stent geeignet ist. Dazu ist jeweils ein Konnektor an einem ersten Ende mit einem Diagonalelement einer ersten Stützstruktur verbunden und an einem zweiten Ende mit einem Diagonalelement einer zweiten Stützstruktur. Zwei aufeinanderfolgende Stützstrukturen können auch über mehr als einen Konnektor miteinander verbunden sein. Einer, mehrere oder alle Konnektoren des erfindungsgemäßen Stents können eine langgestreckte Form mit zwei entgegengesetzten Enden aufweisen. Die Konnnektoren können aus Streben aus einem Implantatwerkstoff gebildet sein. Bevorzugt sind die Konnektoren nur so lang, dass eine ausreichende Flexibilität der beiden benachbarten Stützstrukturen gewährleistet ist, aber nicht so lang, dass der erfindungsgemäße Stent torsionsweich wird. Einer, mehrere oder alle Konnektoren eines erfindungsgemäßen Stents können eine geschwungene Form aufweisen. Einer, mehrere oder alle Konnektoren eines erfindungsgemäßen Stents können jeweils in einem spitzen Winkel vom Diagonalelement abzweigen. Die Konnektoren sind im Wesentlichen in längsachsialer Richtung zwischen den beiden zu verbindenden umlaufenden Stützstrukturen ausgerichtet, wobei die Konnektoren nicht notwendigerweise in paralleler Ausrichtung zur longitudinalen Achse vorliegen.

Der erfindungsgemäße Stent weist einen oder mehrere schlitzförmige Durchbrüche auf, die mit einem röntgendichten Material gefüllt sind. Dabei können diese schlitzförmigen Durchbrüche grundsätzlich an jeder Stelle des Grundkörpers des Stents angebracht sein.

Insbesondere weisen eine, mehrere oder alle Stützstrukturen und/oder Konnektoren des Grundkörpers des Stents einen oder mehrere schlitzförmige Durchbrüche auf. Die schlitzförmigen Durchbrüche erstrecken sich von der dem Lumen des Grundkörpers zugewandten Außenoberfläche der Stützstruktur und/oder des Konnektors durch die ganze Strebe bis einschließlich der Außenoberfläche der Stützstruktur und/oder des Konnektors, die dem Lumen des Stents abgewandt ist. Der schlitzförmige Durchbruch durchdringt die Strebe der Stützstruktur oder des Konnektors also vollständig. Dabei dehnt sich der Durchbruch parallel zur Außenoberfläche der Stützstruktur oder des Konnektors schlitzförmig aus und liegt in dieser Ebene als längliche Öffnung vor, die zwei gegenüberliegende Enden aufweist. Der Abstand der beiden gegenüberliegenden Enden bestimmt die maximale Länge l der Öffnung. Der Abstand der beiden, die gegenüberliegenden Enden verbindenden Wände an der breitesten Stelle der Öffnung bestimmt die maximale Breite b. Dabei weist die längliche Öffnung des schlitzförmigen Durchbruchs bevorzugt ein Verhältnis der maximalen Länge l zur maximalen Breite *b* der Öffnung von ≥ 2 auf, besonders bevorzugt von ≥ 5, ganz besonders bevorzugt von≥10.

Der schlitzförmige Durchbruch weist über die maximale Länge l der länglichen Öffnung eine Krümmung auf, die mindestens zu einem Ende des schlitzförmigen Durchbruchs hin zunimmt. Dadurch ist sichergestellt, dass die Krümmung nicht über die gesamte längliche Öffnung des schlitzförmigen Durchbruchs gleichförmig ist, sondern der schlitzförmige Durchbruch Bereiche mit unterschiedlicher Krümmung aufweist. Dadurch wird eine Spannung erzeugt, die zu einer verbesserten, mechanischen Fixierung des röntgendichten Materials im schlitzförmigen Durchbruch führt.

Eine weitere Verbesserung der mechanischen Fixierung des röntgendichten Materials im schlitzförmigen Durchbruch kann dadurch erreicht werden, dass der schlitzförmige Durchbruch eine Krümmung aufweist, die zu beiden gegenüberliegenden Enden der länglichen Öffnung des schlitzförmigen Durchbruchs hin zunimmt. Unter einer Zunahme wird hier jede Abweichung hin zu einem größeren Zahlenwert verstanden unabhängig vom Vorzeichen (und damit der Richtung) der jeweiligen Krümmung. Die Richtung der jeweiligen Krümmung an den beiden gegenüberliegenden Enden kann gleich oder unterschiedlich sein. Insbesondere kann die Richtung der Krümmung über den Verlauf der maximalen Länge l der länglichen Öffnung des schlitzförmigen Durchbruchs mehrmals wechseln. So kann der Verlauf der Krümmung beispielsweise einen mäanderförmig ausgeprägt sind.

In einer bevorzugten Ausführungsform verläuft die Krümmung im Bereich der beiden gegenüberliegenden Enden der länglichen Öffnung des schlitzförmigen Durchbruchs im Wesentlichen gleichförmig oder gegenläufig. Insbesondere kann die Krümmung derart verlaufen, dass die Form der länglichen Öffnung und bevorzugt die Ausbildung der gegenüberliegenden Enden, eine Achsensymmetrie oder eine Punktsymmetrie aufweist.

Der Verlauf der Krümmung kann insbesondere so gewählt sein, dass die längliche Öffnung des schlitzförmigen Durchbruchs im Bereich der gegenüberliegenden Enden bogen-, haken- oder schneckenförmig ausgebildet ist. Bevorzugte Ausführungen sind beispielhaft in den Figuren 1 bis 7 schematisch dargestellt. In Figur 1 sind die beiden gegenüberliegendnen Enden als gleichförmige Haken ausgebildet. Die längliche Öffnung ist in diesem Fall achsensymmetrisch zur Schnittebene A - A. In Figur 2 sind die beiden gegenüberliegenden Enden als gegenläufige Haken ausgebildet. Wie in Figur 3 gezeigt, kann ein schlitzförmiger Durchbruch auch nur an einem Ende einen entsprechenden Krümmungsverlauf aufweisen. Dabei kann ein Bestandteil des Grundkörpers des Stents mehr als einen schlitzförmigen Durchbruch aufweisen, wobei die schlitzförmigen Durchbrüche gleichförmig (Figur 3) oder gegenläufig (Figur 4) ausgebildet sein können. Die Bestandteile des Grundkörpers können für die Aufnahme eines schlitzförmigen Durchbruchs in ihrer Form spezifisch angepasst und/oder ausgebildet sein. So weist der Bestandteil des Grundgerüsts des Stents in Figur 5 einen Fortsatz auf, der der Aufnahme eines schlitzförmigen Durchbruchs in Form einer Schnecke dient. In den Figuren 6 und 7 sind alternative Ausführungen der Grundgerüstgeometrie gezeigt, die spezifisch an die Aufnahme von schlitzförmigen Durchbrüchen angepasst sind.

Der schlitzförmige Durchbruch des erfindungsgemäßen Stents ist mit einem röntgendichten Material gefüllt. Das röntgendichte Material zeichnet sich dadurch aus, dass es in einer bildgebenden Röntgenuntersuchung darstellbar ist. Hier können röntgendichte Materialien verwendet werden, wie sie in bekannten Röntgenmarkern zum Einsatz kommen. Insbesondere enthält oder besteht das röntgendichte Material aus Au, Pt und/oder Ta oder eine Legierung oder Mischung davon. Dem Fachmann sind geeignete röntgendichte Materialien bekannt.

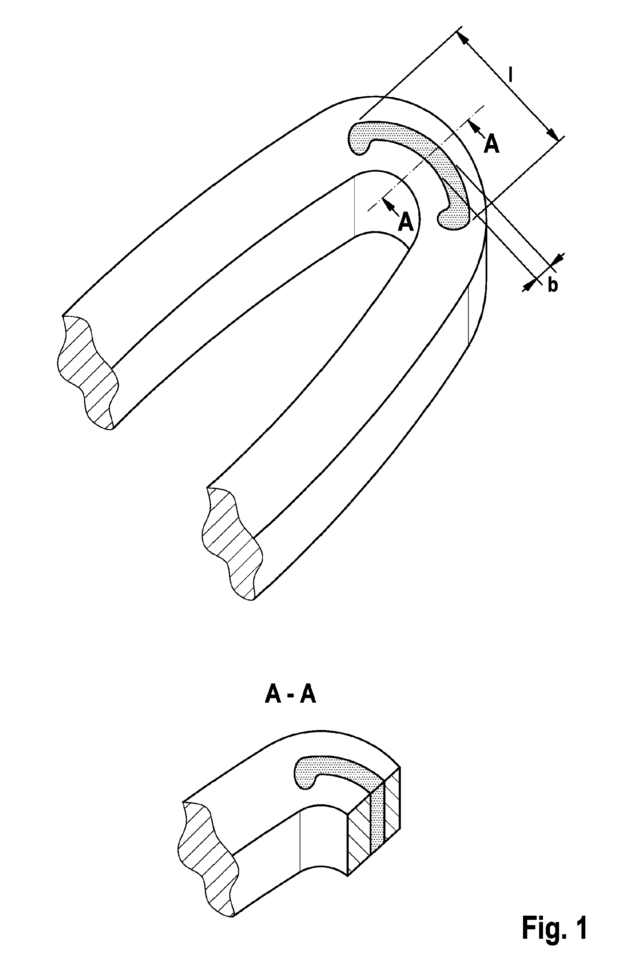

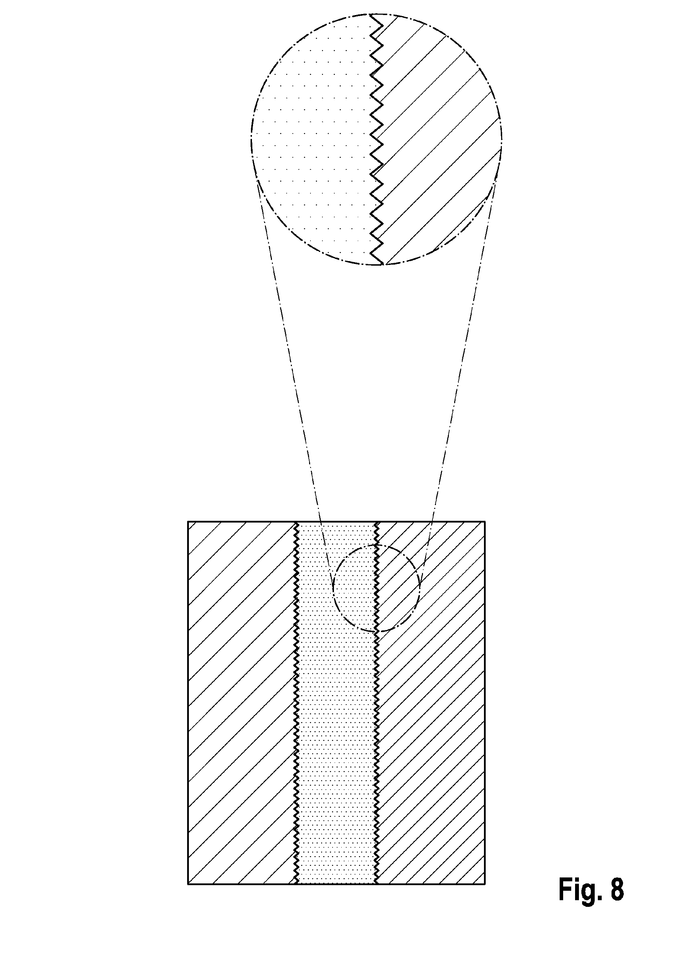

Da im erfindungsgemäßen Stent das röntgendichte Material über eine relativ große Oberfläche des schlitzförmigen Durchbruchs mit dem Grundkörper des Stents in Kontakt steht und zusätzlich die Wahl der Krümmung eine mechanische Fixierung des röntgendichten Materials im Durchbruch unterstützt, kann die Füllung mit dem röntgendichten Material mit der Außenoberfläche des jeweiligen Bestandteils des Grundkörpers des Stents einen ebenen Abschluss bilden. Dies hat den Vorteil, dass weder in das Lumen des Stents noch auf der mit der Gefäßwand zu kontaktierenden Oberfläche des Grundkörpers des Stents Überstände von röntgendichtem Material hinausragen und dort zu Verwirbelungen, Verletzungen oder anderen Störungen führen können. Die Nutzoberflächen des Grundkörpers bleiben eben, während eine ausreichende Fixierung des röntgendichten Materials im schlitzförmigen Durchbruch gewährleistet ist. In Figur 8 ist schematisch eine Draufsicht auf die Schnittfläche der Schnittebene entlang der Schnittführung A - A aus Figur 1 gezeigt. Dabei ist zu erkennen, dass die Füllung mit dem röntgendichten Material auf beiden Seiten des schlitzförmigen Durchbruchs derart mit der Außenoberfläche des Bestandteils des Grundgerüsts abschließt, dass sich eine ebene Außenfläche ergibt. Durch die Oberflächenrauhigkeit der Innenwände des schlitzförmigen Durchbruchs erfolgt eine formschlüssige Fixierung der Füllung mit röntgendichtem Material im schlitzförmigen Durchbruch.

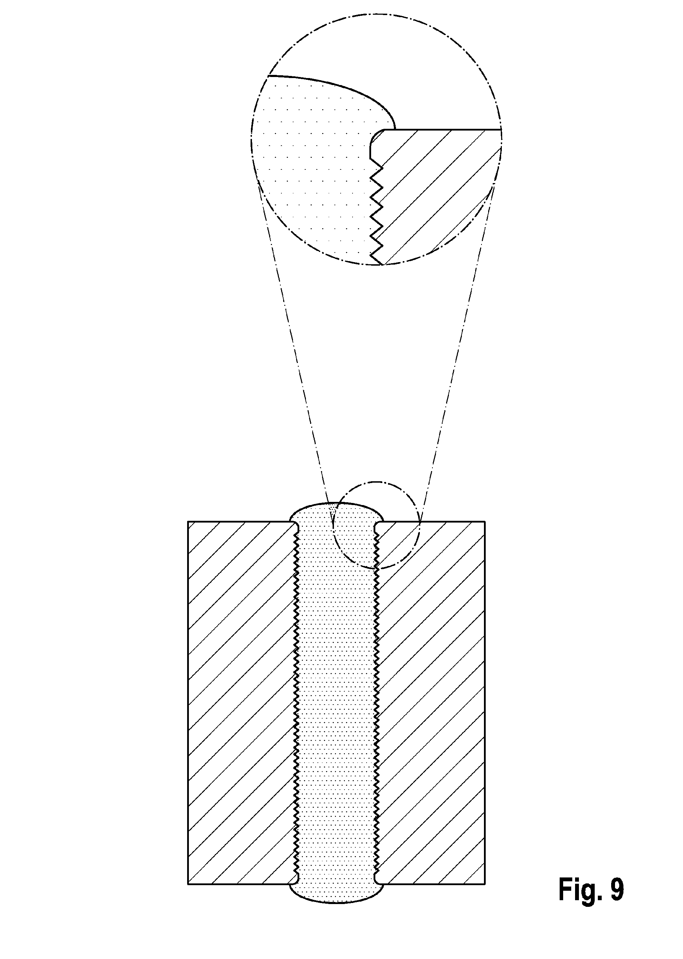

Alternativ dazu kann die Füllung mit einem röntgendichten Material mit der den schlitzförmigen Durchbruch umgebenden Außenoberfläche der Stützstruktur und/oder des Konnektors einen konvexen Abschluss bilden. Dadurch kann die mechanische Fixierung des röntgendichten Materials im Grundkörper des Stents noch weiter verstärkt werden. Bevorzugt kann der konvexe Abschluss über die Kante der Außenoberfläche überstehend ausgebildet sein. Besonders bevorzugt sind die Kanten der Außenoberfläche der Stützstruktur und/oder des Konnektors abgerundet gestaltet, so dass der Überstand der Füllung über die Fläche der abgerundeten Kanten mit der Außenoberfläche in Kontakt tritt. Dies hat den Vorteil, dass die Wahrscheinlichkeit eines Bruches des Überstandes an der Kante der Außenoberfläche geringer ist. Eine entsprechende Ausführung ist beispielhaft in Figur 9 dargestellt. Dabei erfolgt die formschlüssige Fixierung nicht nur über die Rauhigkeit der Innenoberflächen des schlitzförmigen Durchbruchs, sondern zusätzlich über die auf beiden Seiten ausgebildeten Überstände über den abgerundeten Kanten der Außenoberflächen der Stützstruktur und/oder des Konnektors.

Der Grundkörper des erfindungsgemäßen Stents kann aus einem Implantatwerkstoff geformt sein. Ein Implantatwerkstoff ist ein nicht lebendes Material, das für eine Anwendung in der Medizin eingesetzt wird und mit biologischen Systemen in Wechselwirkung tritt. Grundvoraussetzungen für den Einsatz eines Werkstoffes als Implantatwerkstoff, der bei bestimmungsgemäßen Gebrauch mit der Körperumgebung in Kontakt steht, ist dessen Körperverträglichkeit (Biokompatibilität). Unter Biokompatibilität wird die Fähigkeit eines Werkstoffes verstanden, in einer spezifischen Anwendung eine angemessene Gewebereaktion hervorzurufen. Dies beinhaltet eine Anpassung der chemischen, physikalischen, biologischen und morphologischen Oberflächeneigenschaften eines Implantates an das Empfängergewebe mit dem Ziel einer klinisch erwünschten Wechselwirkung. Die Biokompatibilität des Implantatswerkstoffs ist weiterhin abhängig vom zeitlichen Ablauf der Reaktion des Biosystems in das implantiert wird. So treten relativ kurzfristig Reizungen und Entzündungen auf, die zu Gewebeveränderungen führen können. Biologische Systeme reagieren demnach in Abhängigkeit von den Eigenschaften des Implantatswerkstoffs in verschiedener Weise. Entsprechend der Reaktion des Biosystems können die Implantatswerkstoffe in bioaktive, bioinerte und biokorrodierbare/resorbierbare Werkstoffe unterteilt werden.

Der Grundkörper des erfindungsgemäßen Stents kann aus jedem Implantatwerkstoff bestehen, der für die Herstellung von Implantaten, insbesondere Stens, geeignet ist. Implantatwerkstoffe für Stents umfassen Polymere, metallische Werkstoffe und keramische Materialien. Biokompatible Metalle und Metalllegierungen für Permanentimplantate beinhalten beispielsweise rostfreie Stähle (zum Beispiel 316L), Kobaltbasislegierungen (zum Beispiel CoCrMo-Gußlegierungen, CoCrMo-Schmiedelegierungen, CoCrWNi-Schmiedelegierungen und CoCrNiMo-Schmiedelegierungen), Reintitan und Titanlegierungen (z. B. cp Titan, TiAl6V4 oder TiAl6Nb7) und Goldlegierungen.

Bevorzugt weist der Grundkörper einen metallischen Implantatwerkstoff auf oder besteht daraus.

Besonders bevorzugt weist der erfindungsgemäße Stent einen Grundkörper auf, der einen biodegradierbaren Implantatwerkstoff enthält oder daraus besteht. Im Bereich biokorrodierbarer Stents wird der Einsatz von Magnesium oder Reineisen sowie biokorrodierbaren Basislegierungen der Elemente Magnesium, Eisen, Zink, Molybdän und Wolfram vorgeschlagen. Insbesondere kann der Grundkörper eines erfindungsgemäßen Stents eine biokorrodierbare Magnesiumlegierung aufweisen oder daraus bestehen.

Unter Legierung wird vorliegend ein metallisches Gefüge verstanden, deren Hauptkomponente Magnesium, Eisen, Zink oder Wolfram ist. Hauptkomponente ist die Legierungskomponente, deren Gewichtsanteil an der Legierung am höchsten ist. Ein Anteil der Hauptkomponente beträgt vorzugsweise mehr 50 Gew.%, insbesondere mehr als 70 Gew.%.

Die Legierungen der Elemente Magnesium, Eisen, Zink oder Wolfram können so in ihrer Zusammensetzung gewählt sein, dass sie biokorrodierbar sind. Als biokorrodierbar im Sinne der Erfindung werden Legierungen bezeichnet, bei denen in physiologischer Umgebung ein Abbau stattfindet, der letztendlich dazu führt, dass das gesamte Implantat oder der aus dem Werkstoff gebildete Teil des Implantates seine mechanische Integrität verliert. Als Prüfmedium zur Testung des Korrosionsverhaltens einer in Frage kommenden Legierung dient künstliches Plasma, wie es nach EN ISO 10993-15:2000 für Biokorrosionsuntersuchungen vorgeschrieben ist (Zusammensetzung NaCl 6,8 g/l, CaCl₂ 0,2 g/l, KCl 0,4 g/l, MgSO₄ 0,1 g/l, NaHCO₃ 2,2 g/l, Na₂HPO₄ 0,126 g/l, NaH₂PO₄ 0,026 g/l). Eine Probe der zu untersuchenden Legierung wird dazu in einem verschlossenen Probenbehälter mit einer definierten Menge des Prüfmediums bei 37°C gelagert. In zeitlichen Abständen - abgestimmt auf das zu erwartende Korrosionsverhalten - von wenigen Stunden bis zu mehreren Monaten werden die Proben entnommen und in bekannter Weise auf Korrosionsspuren untersucht. Das künstliche Plasma nach EN ISO 10993-15:2000 entspricht einem blutähnlichen Medium und stellt damit eine Möglichkeit dar, eine physiologische Umgebung im Sinne der Erfindung reproduzierbar nachzustellen.

Der Begriff Korrosion bezieht sich vorliegend auf die Reaktion eines metallischen Werkstoffes mit seiner Umgebung, wobei eine messbare Veränderung des Werkstoffs bewirkt wird, die ― bei Einsatz des Werkstoffs in einem Bauteil - zu einer Beeinträchtigung der Funktion des Bauteils führt. Ein Korrosionssystem besteht vorliegend aus dem korrodierenden metallischen Werkstoff sowie einem flüssigen Korrosionsmedium, das in seiner Zusammensetzung die Verhältnisse in physiologischer Umgebung nachstellt oder ein physiologisches Medium, insbesondere Blut ist. Werkstoffseitig beeinflussen die Korrosion Faktoren, wie die Zusammensetzung und Vorbehandlung der Legierung, mikro- und submikroskopische Inhomogenitäten, Randzoneneigenschaften, Temperatur- und Spannungszustand und insbesondere die Zusammensetzung einer die Oberfläche bedeckenden Schicht. Auf Seiten des Mediums wird der Korrosionsprozess durch Leitfähigkeit, Temperatur, Temperaturgradienten, Azidität, Volumen-Oberflächen-Verhältnis, Konzentrationsunterschied sowie Strömungsgeschwindigkeit beeinflusst.

In DE 197 31 021 A1 werden geeignete biokorrodierbare metallische Implantatwerkstoffe vorgestellt, deren Hauptbestandteil ein Element aus der Gruppe Alkalimetalle, Erdalkalimetalle, Eisen, Zink und Aluminium ist. Als besonders geeignet werden Legierungen auf Basis von Magnesium, Eisen und Zink beschrieben. Nebenbestandteile der Legierungen können Mangan, Cobalt, Nickel, Chrom, Kupfer, Cadmium, Blei, Zinn, Thorium, Zirkonium, Silber, Gold, Palladium, Platin, Silizium, Calcium, Lithium, Aluminium, Zink und Eisen sein. Weiterhin ist aus der DE 102 53 634 A1 der Einsatz einer biokorrodierbaren Magnesiumlegierung mit einem Anteil von Magnesium >90%, Yttrium 3,7 ― 5,5%, Seltenerdmetallen 1,5 ― 4,4% und Rest < 1% bekannt, die sich insbesondere zur Herstellung eines Stents, z. B. in Form eines selbstexpandierenden oder ballonexpandierbaren Stent, eignet.

Der erfindungsgemäße Stent kann z. B. dadurch hergestellt werden, dass nach Herstellung des Stentgrundkörpers die schlitzförmigen Durchbrüche in der gewünschten Krümmung und Form in den Grundkörper eingearbeitet werden, beispielsweise mittels Laserschnitt-oder Laserfräsverfahren. Anschließend kann das röntgendichte Material aufgebracht werden. Dies kann beispielsweise durch Abscheidungsverfahren geschehen, wobei solange röntgendichtes Material abgeschieden wird, bis die schlitzförmigen Durchbrüche vollständig mit röntgendichtem Material aufgefüllt sind. Anschließend kann überschüssiges röntgendichtes Material vom Grundkörper des Stents wieder entfernt werden.

Im Folgenden wird die Erfindung anhand von Ausführungsbeispielen näher erläutert.

Figuren:
- Fig. 1: zeigt schematisch einen Ausschnitt des Grundgerüsts eines erfindungsgemäßen Stents, wobei der dargestellte Bestandteil des Grundgerüsts im Bereich des Bogenelementes einen schlitzförmigen Durchbruch aufweist, der mit röntgendichtem Material gefüllt ist, wobei die beiden gegenüberliegenden Enden des schlitzförmigen Durchbruchs als Haken mit gleichförmigem Krümmungsverlauf ausgebildet sind. Im Schnitt entlang der Schnittebene A - A wird sichtbar, dass der schlitzförmige Durchbruch die Strebe des Bogenelementes vollständig durchdringt.

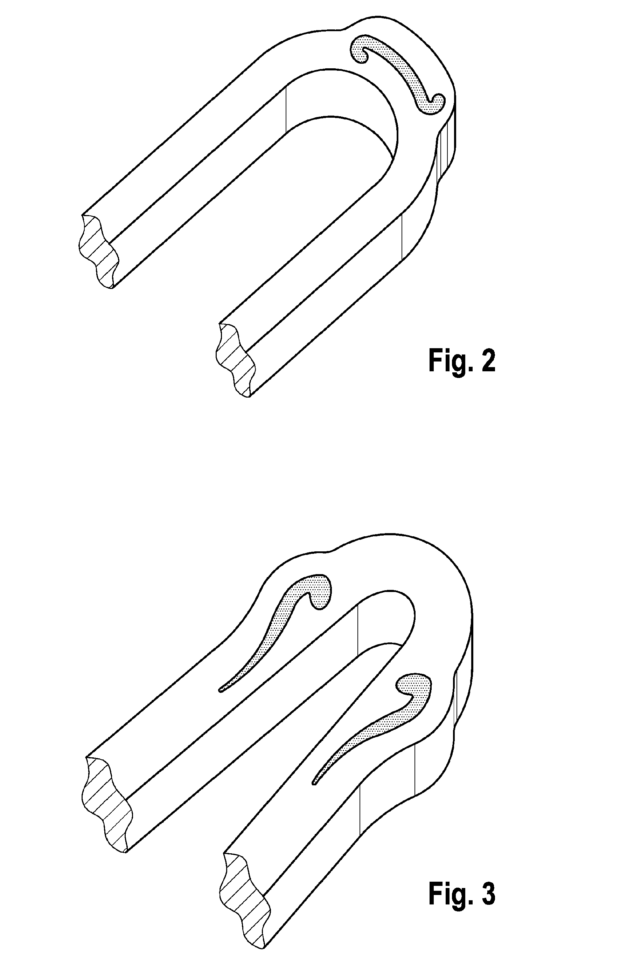

- Fig. 2: zeigt schematisch einen Ausschnitt des Grundgerüsts eines erfindungsgemäßen Stents, wobei der dargestellte Bestandteil des Grundgerüsts im Bereich des Bogenelementes einen schlitzförmigen Durchbruch aufweist, der mit röntgendichtem Material gefüllt ist, wobei die beiden gegenüberliegenden Enden des schlitzförmigen Durchbruchs als Haken mit gegenläufigem Krümmungsverlauf ausgebildet sind.
- Fig. 3: zeigt eine weitere Ausführungsform des erfindungsgemäßen Stents, wobei der dargestellte Bestandteil des Grundgerüsts zwei schlitzförmige Durchbrüche aufweist. Jeder der beiden schlitzförmigen Durchbrüche weist nur im Bereich eines der beiden Enden einen entsprechenden hakenförmigen Krümmungsverlauf auf, wobei der Krümmungsverlauf der beiden schlitzförmigen Durchbrüche gleichförmig ist.

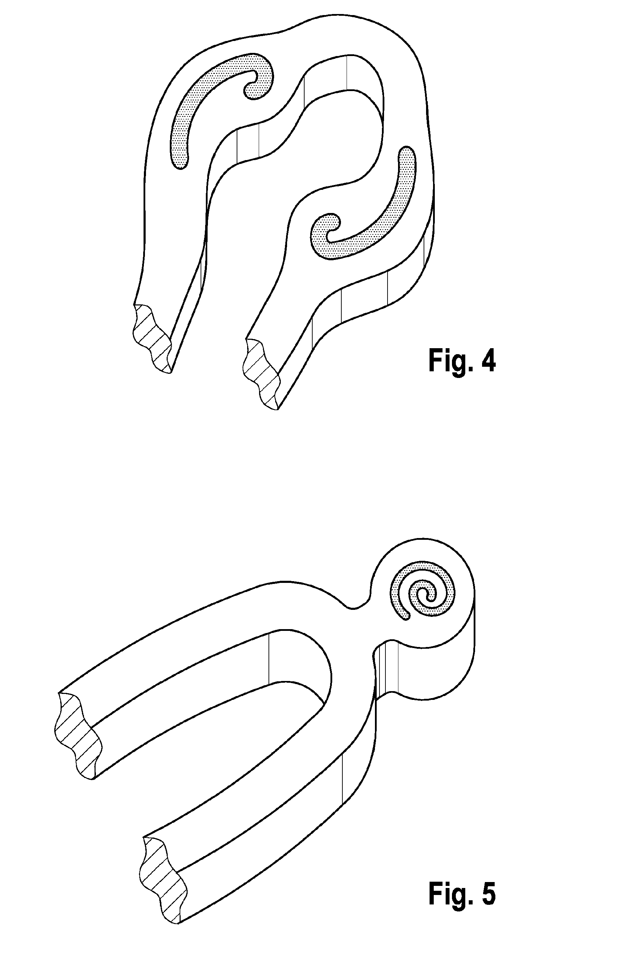

- Fig. 4: zeigt eine weitere Ausführungsform des erfindungsgemäßen Stents, wobei der dargestellte Bestandteil des Grundgerüsts zwei schlitzförmige Durchbrüche aufweist. Jeder der beiden schlitzförmigen Durchbrüche weist nur im Bereich eines der beiden Enden einen entsprechenden hakenförmigen Krümmungsver- lauf auf, wobei der Krümmungsverlauf der beiden schlitzförmigen Durchbrüche gegenläufig ist.
- Fig. 5: zeigt schematisch einen Ausschnitt des Grundgerüsts eines erfindungsgemäßen Stents, wobei der dargestellte Bestandteil des Grundgerüsts im Bereich des Bogenelementes einen Fortsatz mit einem schlitzförmigen Durchbruch aufweist, der mit röntgendichtem Material gefüllt ist, wobei der schlitzförmige Durchbruch als Schnecke ausgebildet ist.

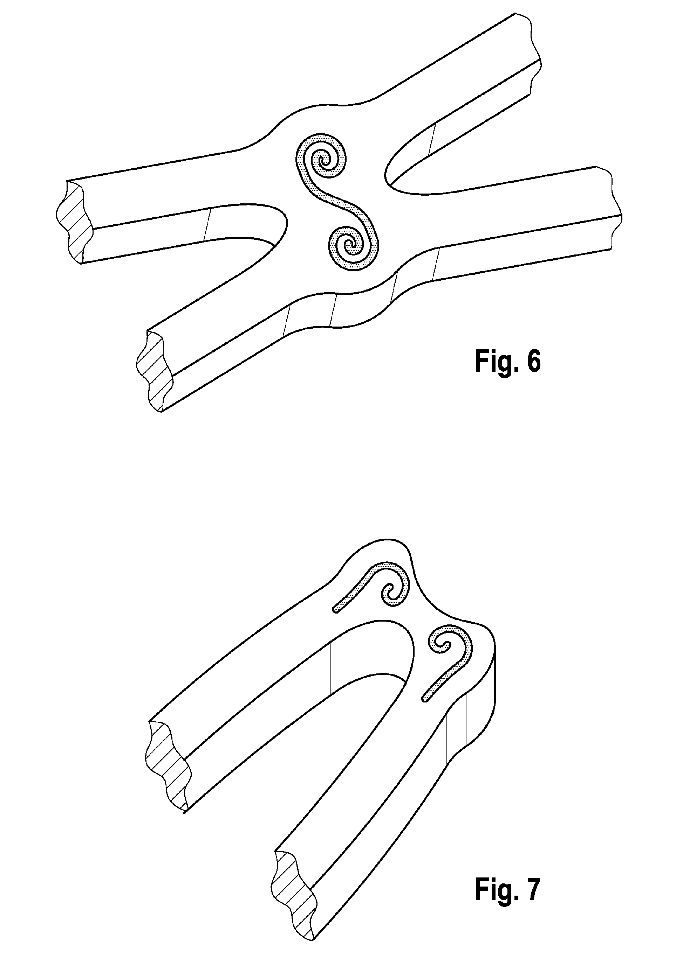

- Fig. 6: zeigt schematisch einen Ausschnitt des Grundgerüsts eines erfindungsgemäßen Stents, wobei der dargestellte Bestandteil des Grundgerüsts einen verdickten Bereich mit einem schlitzförmigen Durchbruch aufweist, der mit röntgendichtem Material gefüllt ist, wobei die beiden gegenüberliegenden Enden des schlitzförmigen Durchbruchs als gegenläufige Schnecken ausgebildet sind.
- Fig. 7: zeigt eine weitere Ausführungsform des erfindungsgemäßen Stents, wobei der dargestellte Bestandteil des Grundgerüsts zwei Verdickungen mit jeweils einem schlitzförmigen Durchbruch aufweist. Jeder der beiden schlitzförmigen Durchbrüche weist nur im Bereich eines der beiden Enden einen entsprechenden hakenförmigen Krümmungsverlauf auf, wobei der Krümmungsverlauf der beiden schlitzförmigen Durchbrüche gleichförmig ist.
- Fig. 8: zeigt eine schematische Draufsicht auf die Schnittfläche der Schnittebene entlang der Schnittführung A - A aus Figur 1. Dabei ist zu erkennen, dass die Füllung mit dem röntgendichten Material auf beiden Seiten des schlitzförmigen Durchbruchs derart mit der Außenoberfläche des Bestandteils des Grundgerüsts abschließt, dass sich eine ebene Außenfläche ergibt. Durch die Oberflächenrauhigkeit der Innenwände des schlitzförmigen Durchbruchs erfolgt eine formschlüssige Fixierung der Füllung mit röntgendichtem Material im schlitzförmigen Durchbruch.
- Fig. 9: zeigt eine schematische Draufsicht auf die Schnittfläche der Schnittebene entlang der Schnittführung A - A aus Figur 1. Dabei erfolgt die formschlüssige Fixierung der Füllung mit dem röntgendichten Material nicht nur über die Rauhigkeit der Innenoberflächen des schlitzförmigen Durchbruchs, sondern zusätzlich über die auf beiden Seiten ausgebildeten Überstände der Füllung über den abgerundeten Kanten der Außenoberflächen der Stützstruktur und/oder des Konnektors.

### Ausführungsbeispiele:

### Ausführungsbeispiel 1:

In einem handelsüblichen Stent werden in die Streben des Grundkörpers mittels Laserschneidverfahren schlitzförmige Durchbrüche eingearbeitet. Der Stent mit den schlitzförmigen Durchbrüchen wird anschließend elektropoliert und ultraschallgereinigt zur Vorbereitung der galvanische Abscheidung von z. B. Pt, Au, (ggf. Ta per ionischer Flüssigkeit). Mittels einer galvanischen Prozessabfolge - Haftgoldprozess, Deckgold - werden die schlitzförmigen Durchbrüche sowie die flächige Umgebung der Streben mit dem Markermaterial z. B. Au, Pt, Ta durch zugeschnittene Prozessparameter beschichtet, mit dem Ergebnis, dass der schlitzförmige Durchbruch vollständig mit dem Markermaterial haftfest und formschlüssig gefüllt ist.

Die aufgefüllten schlitzförmigen Durchbrüche können anschließend lithografisch abgedeckt und/oder geschützt werden für einen nachfolgen Prozessschritt "Strippen", durch den das röntgendichte Material außerhalb des schlitzförmigen Bereichs in Lösung geht und somit wieder abgetragen wird.

Im Ergebnis verbleibt ein Stent mit einem röntgendichten Marker, der haftfest und zudem formschlüssig hohen mechanischen Belastungen ausgesetzt werden kann, ohne dass Mikrorisse oder Ablösungen entstehen.

### Ausführungsbeispiel 2:

In einem handelsüblichen Stent werden in die Streben des Grundkörpers mittels Laserschneidverfahren schlitzförmige Durchbrüche eingearbeitet. Der Stent mit den schlitzförmigen Durchbrüchen wird anschließend elektropoliert und ultraschallgereinigt zur Vorbereitung der galvanische Abscheidung von z. B. Pt, Au, (ggf. Ta per ionischer Flüssigkeit). Mittels einer galvanischen Prozessabfolge - Haftgoldprozess, Deckgold - werden die schlitzförmigen Durchbrüche sowie die flächige Umgebung der Streben mit dem Markermaterial z. B. Au, Pt, Ta durch zugeschnittene Prozessparameter beschichtet, mit dem Ergebnis, dass der schlitzförmige Durchbruch vollständig mit dem Markermaterial haftfest und formschlüssig gefüllt ist.

### Der Prozessschritt "Strippen" entfällt.

Im Ergebnis verbleibt ein Stent mit einem röntgendichten Marker, der haftfest und zudem formschlüssig hohen mechanischen Belastungen ausgesetzt werden kann, ohne dass Mikrorisse oder Ablösungen entstehen.

### Ausführungsbeispiel 3:

Die Segmente der beidseitigen Stentenden werden lithografisch mit UV/Laser reaktivem Schutzlack abgedeckt. Die Areale der Stentenden an denen schlitzförmige Durchbrüche eingefügt werden sollen, werden per UV bzw. Laserbelichtung mit einem nachfolgenden Prozessschritt "Entwicklung" freigelegt und gereinigt. Die freigelegten, gereinigten schlitzförmigen Durchbrüche können nun galvanotechnisch in einem Beschichtungsverfahren mit dem röntgendichten Material vollständig aufgefüllt werden. In Abhängigkeit von den Prozessparametem kann die stirnseitige Oberfläche der Füllung der schlitzförmigen Durchbrüche konvex, konkav oder bündig zur Außenoberfläche der umgebenden Streben aufgebaut werden

Im Ergebnis verbleibt ein Stent mit röntgendichten Markern mit angepassten topologischen Übergängen, ohne scharfe Kanten zur Umgebung gleichen Niveaus , der haftfest und zudem formschlüssig hohen mechanischen Belastungen ausgesetzt werden kann, ohne dass Mikrorisse oder Ablösungen entstehen.

### Ausführungsbeispiel 4:

Der schlitzförmige Durchbruch kann in vielen unterschiedlichen Formen und Varianten verwirklicht werden. So kann der schlitzförmige Durchbruch, wie in Figur 1 gezeigt, beispielsweise derart ausgebildet sein, dass die beiden gegenüberliegenden Enden als Haken mit jeweils gleichförmigem Krümmungsverlauf ausgebildet sind. In der Darstellung des Schnitts entlang der Schnittführung A - A wird deutlich, dass sich der schlitzförmige Durchbruch durch die ganze Strebe erstreckt und diese dabei vollständig durchdringt. Dabei weist der schlitzförmige Durchbruch in der Draufsicht eine längliche Öffnung auf. Die längliche Öffnung des schlitzförmigen Durchbruchs weist ein Verhältnis der maximalen Länge l zur maximalen Breite *b* von ≥ 2 auf.

In den Figuren 2 bis 7 sind weitere beispielhafte Ausführungsformen von alternativen Formen und Varianten offenbart, die ein schlitzförmiger Durchbruch im erfindungsgemäßen Stent annehmen kann.

## Patentansprüche

1. Stent umfassend einen röhrenförmigen Grundkörper mit einem Lumen entlang einer Längsachse, wobei der Grundkörper eine Mehrzahl von umlaufenden Stützstrukturen und einen oder mehrere Konnektoren aufweist, wobei zwei aufeinanderfolgende umlaufende Stützstrukturen über mindestens einen Konnektor miteinander verbunden sind, **dadurch gekennzeichnet, dass** eine, mehrere oder alle Stützstrukturen und/oder Konnektoren einen schlitzförmigen Durchbruch aufweisen, wobei der schlitzförmige Durchbruch mit einem röntgendichten Material gefüllt ist und eine Krümmung aufweist, die mindestens zu einem Ende des schlitzförmigen Durchbruchs hin zunimmt.

2. Stent nach Anspruch 1, wobei der schlitzförmige Durchbruch die dem Lumen des Stents zu- und abgewandte Außenoberfläche der Stützstruktur oder des Konnektors durchdringt und sich parallel zur Außenoberfläche der Stützstruktur oder des Konnektors als längliche Öffnung mit zwei gegenüberliegenden Enden erstreckt.

3. Stent nach Anspruch 2, wobei die längliche Öffnung des schlitzförmigen Durchbruchs ein Verhältnis der maximalen Länge l zur maximalen Breite *b* von ≥ 2 aufweist.

4. Stent nach einem der vorhergehenden Ansprüche, wobei der schlitzförmige Durchbruch eine Krümmung aufweist, die zu beiden gegenüberliegenden Enden des schlitzförmigen Durchbruchs hin zunimmt.

5. Stent nach Anspruch 4, wobei die Richtung der Krümmung der beiden gegenüberliegenden Enden gleich oder unterschiedlich ist.

6. Stent nach Anspruch 5, wobei die Richtung der Krümmung über den Verlauf des schlitzförmigen Durchbruchs mehrfach wechselt, bevorzugt einen mäanderförmigen Verlauf annimmt.

7. Stent nach einem der Ansprüche 4 bis 6, wobei der Verlauf der Krümmung an den beiden gegenüberliegenden Enden des schlitzförmigen Durchbruchs im Wesentlichen gleichförmig oder gegenläufig ist.

8. Stent nach einem der vorhergehenden Ansprüche, wobei der Verlauf der Krümmung an einem oder an den beiden gegenüberliegenden Enden des schlitzförmigen Durchbruchs bogen-, haken- oder schneckenförmig ausgebildet ist.

9. Stent nach einem der vorhergehenden Ansprüche, wobei die Füllung mit einem röntgendichten Material mit der den schlitzförmigen Durchbruch umgebenden Außenoberfläche der Stützstruktur und/oder des Konnektors einen ebenen Abschluss bildet.

10. Stent nach einem der Ansprüche 1 bis 8, wobei die Füllung mit einem röntgendichten Material mit der den schlitzförmigen Durchbruch umgebenden Außenoberfläche der Stützstruktur und/oder des Konnektors einen konvexen Abschluss bildet, bevorzugt der konvexe Abschluss ist über die abgerundete Kante der Außenoberfläche überstehend ausgebildet, besonders bevorzugt sind die Kanten der Außenoberfläche der Stützstruktur und/oder des Konnektors abgerundet.

11. Stent nach einem der vorhergehenden Ansprüche, wobei das röntgendichte Material Au, Pt, Ta und/oder eine Mischung oder Legierung davon enthält oder daraus besteht.

12. Stent nach einem der vorhergehenden Ansprüche, wobei der Grundkörper einen metallischen Implantatwerkstoff aufweist oder daraus besteht.

13. Stent nach einem der vorhergehenden Ansprüche, wobei der Grundkörper einen biokorrodierbaren Implantatwerkstoff aufweist oder besteht.

14. Stent nach einem der vorhergehenden Ansprüche, wobei der Grundkörper eine biokorrodierbare Magnesiumlegierung aufweist oder daraus besteht.
